(19) Europäisches Patentamt
European Patent Office
Office européen des brevets

(11) Publication number: **0 215 038**
**B1**

(12) # EUROPEAN PATENT SPECIFICATION

(45) Date of publication of patent specification: **09.08.89**

(51) Int. Cl.[4]: **C 07 H 1/00**, C 07 H 13/02, C 07 H 13/04, C 07 H 9/04, C 07 D317/24, C 07 D317/64, C 12 P 7/62, C 12 P 17/04, C 12 P 19/02

(21) Application number: **86901364.9**

(22) Date of filing: **27.02.86**

(86) International application number: **PCT/DK 86/00017**

(87) International publication number: **WO/8605186 (12.09.86 Gazette 86/20)**

(54) **PROCESS FOR ESTERIFICATION.**

(30) Priority: **27.02.85 DK 877/85**
**07.11.85 DK 5147/85**

(43) Date of publication of application: **25.03.87 Bulletin 87/13**

(45) Publication of the grant of the patent: **09.08.89 Bulletin 89/32**

(84) Designated contracting states: **AT BE CH DE FR GB IT LI LU NL SE**

(56) References cited:
EP-A- 0 018 342
EP-A- 0 061 023
EP-A- 0 126 416
DE-A- 1 543 495
DE-A- 2 233 245
DE-B- 1 183 895
GB-A- 1 477 880
GB-A- 1 589 916
US-A- 3 251 827
US-A- 3 686 238
**The file contains technical information submitted after the application was filed and not included in this specification**

(73) Proprietor: **NOVO INDUSTRI A/S**
**Novo Allé**
**DK-2880 Bagsvaerd (DK)**

(72) Inventor: **GODTFREDSEN, Sven, Erik**
**15 B, Smedegade**
**DK-3500 Vaerlose (DK)**
Inventor: **BUNDGARD, Peter**
**36, Pra estegaardsvej**
**DK-6500 Vojens (DK)**
Inventor: **ANDRESEN, Otto**
**5, Stenlosevej**
**DK-3660 Stenlose (DK)**

(74) Representative: **Brown, John David et al**
**FORRESTER & BOEHMERT Widenmayerstrasse 4/I**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**EP 0 215 038 B1**

## Description

### Background of the invention

The present invention relates to a process for preparing acetal and ketal esters of polyols, and, optionally, monoesters of carbohydrates or monoglycerides.

Formation of acetals and ketals is a well established process within the art of organic chemistry. It is thus well known that aldehydes and ketones may combine with simple alcohols under formation of acetals and ketals. Usually, these reactions are carried out in the presence of an acid catalyst and in a solvent which, by azeotropic distillation, will serve to remove the water generated in the course of the reaction thereby displacing the equilibrium towards acetal or ketal formation.

Formation of carboxylic acid esters is, likewise, a well established organic chemical reaction. This process may be carried out by heating a mixture of a carboxylic acid and an alcohol or by contacting these reagents in the presence of a catalyst. Traditionally, an acid catalyst is often used in this context but, recently, it has been shown that enzymes such as esterases and lipases may be used as catalysts in ester synthesis.

In contrast to the predictable course, the simplicity of the processes and the high yields which thus characterize formation of actetals, ketals and carboxylic acid esters of simple monofunctional alcohols, formation of such derivatives from polyols pose problems. However desirable, it is often difficult to prepare, for example, pure derivatives of polyols and carboxylic acids in a satisfactorily high yield. This difficulty has been clearly experienced during attempts to prepare monocarboxylic acid esters of glycerol — the so-called monoglycerides — which are currently used on a large scale as emulsifying agents. Attempted preparation of these compounds by acid or base catalyzed hydrolysis of readily available, naturally occurring triglycerides invariably provides mixtures of mono-, di- and triglycerides besides free carboxylic acids, glycerol and inorganic salts. This is the case also for even the best, currently applied industrial processes for preparation of monoglycerides which comprises glycerolysis of triglycerides in the presence of a strong base at temperatures above 200 °C. Invariably this process suffers from low yields and formation of by-products which necessitates extensive purification of the desired monoglycerides. Likewise, attempted use of enzymes, for example lipases or esterases, for preparation of monoglycerides suffer from formation of undesired by-products such as glycerol, free carboxylic acids and diglycerides. The origin of these difficulties lies in the polyfunctionality of the glycerol molecule which carries three hydroxyl groupings of comparable reactivity towards esterification, the consequence being that selective esterification of the triol is difficult to achieve as is selective cleavage of ester linkages in triglycerides. This is true, also, in regard to other polyols among which the carbohydrates constitute a particularly important group. It is a quite difficult task to achieve selective, partial esterification of these compounds by chemical as well as by enzymatic means. Moreover, the different hydrophilic and hydrophobic properties of polyols and acyl donors give rise to technical problems in the design of esterification processes. On one hand, polyols of interest are often soluble only in hydrophilic solvents such as water. On the other hand, the acyl donor is often soluble only in an organic solvent. The consequence of this is that the two species are brought in contact only with difficulty and thus normally react in only poor yields to the desired esters.

Danish patent application No. 938/80 describes a process by which glycerol is reacted with a keton and the resulting ketal is esterified using an acid chloride of an acid. In French patent No. 2 147 091 a similar esterification using an acid instead of an acid chloride is described. A similar process is described in U.S. Patent-specification No. 3 686 238. In none of these publications the use of an enzyme is mentioned.

One object of this invention is to provide a process whereby the above problems are overcome.

A further object of this invention is to provide a process whereby the desired compounds are obtained in high yields.

A still further object of this invention is to provide a process wherein the desired compounds are easily obtained.

### Detailed practice of the invention

It has now surprisingly been found that it is possible to overcome the problems associated with hydrophilic properties as well as the polyfunctionality of polyols in a single process thereby making possible selective, partial esterification of polyols of the general formula IIa

$$\begin{array}{r} R^5\text{-CHOH} \\ | \\ \text{CHOH} \\ | \\ R^4\text{-CHOH} \end{array} \qquad \text{(IIa)}$$

wherein $R^4$ and $R^5$ each are as stated below, in high yield by carrying out the esterification process concomitant with ketalisation or acetalisation of the polyols of formula IIa. More particularly, partial esterification of the polyols of formula IIa can be achieved, according to the process of this invention, by treating, under dehydrating conditions or other circumstances which favour ketalisation or acetalisation, a polyol of formula IIa with a carbonyl compound of formula III stated below, or a precursor thereof and a carboxylic acid or a carboxylic acid ester of formula IV stated below, in the presence of an agent which catalyses formation of acetals or ketals and an enzyme which catalyse esterification. Treatment of a polyol of formula IIa with a carbonyl compound of formula III may be carried out simultaneously with treatment of the polyol of formula IIa with a carboxylic acid or a carboxylic acid derivative of formula IV, or treatment with a carbonyl compound of formula III may be carried out alternating with or prior to treatment of the polyol of formula IIa with a carboxylic acid or a carboxylic acid derivative of formula IV. The process of this invention may, if desired, be carried out in a solvent, in a mixture of solvents or in the absence of solvents. Preferably, the process of this invention is carried out in a solvent of a hydrocarbon, for example, pentane, hexane or benzene, or in the absence of a solvent.

The agent catalyzing formation of acetal or ketal and applied in the process of this invention may be any agent known to catalyze formation of acetals and ketals from the corresponding carbonyl compound, for example, acids such as p-toluenesulfonic acid, methansulfonic acid, sulfuric acid, Dowex™-50 or a carboxylic acid, for example, adipic acid, or reagents such as boron trifluoride. Reagents other than free aldehydes and ketones may also be applied for acetal or ketal formation according to the process of this invention.

The enzyme catalyzing esterification and applied in the process of this invention may be an enzyme such as a lipase or an esterase or any other enzyme exhibiting an esterase activity. Enzymes applied according to the process of this invention may be used as the native enzymes, as immobilized enzymes, as chemically modified enzymes or as encapsulated enzymes.

It is very surprising that the enzymes can be used in the acid medium containing the carbonyl compound of formula III and the acetalized or ketalized compound of formula I stated below.

Dehydration applied according to the process of this invention may be achieved by any known means for dehydration. Preferably, dehydration is achieved by azeotropic distillation of water by the solvent applied in the process of the invention. Also, the dehydration can be achieved using an insoluble dehydrating agent, for example a molecular sieve.

Alternatively, the process of this invention may be carried out by reacting an alcohol of the general formula IIb

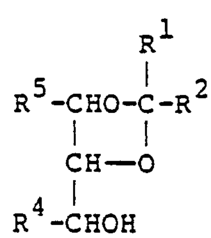

(IIb)

wherein $R^1$, $R^2$, $R^4$ and $R^5$ each are as stated below, with a carboxylic acid or a carboxylic acid ester of formula IV in the presence of an enzyme catalyzing esterification. This process is carried out as described above for the esterification. Compounds of formula IIb can by the process of this invention be used as starting material for preparing the desired esters of carbohydrates or monoglycerides of formula VII. Alcohols of formula IIb can be prepared by reacting a compound of formula IIa with a carbonyl compound of formula III as stated above.

The process of this invention may be carried out batch-wise or continuously at temperatures varying from ambient temperature to the boiling point of the applied solvent or, in the absence of solvents, at temperatures up to about 260 °C. The reaction time may be from a few hours to several days. The products of the reaction may be isolated in a manner known per se, optionally after removal of the applied reagents and solvents in a manner known per se.

According to a preferred embodiment of this invention the esterification with the carboxylic acid or carboxylic acid ester of formula IV is carried out after all the hydroxy groups, apart from only one, are acetalized or ketalized and, thereafter, the hydroxy groups are, if desired, liberated.

The above, two process variants can be combined in that they relate to a process for preparing compounds of the general formula I

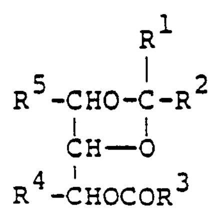

(I)

wherein $R^1$, $R^2$ and $R^3$ are the same or different each representing hydrogen, alkyl, lower alkoxy, aryl or aryl (lower alkyl) each of which may be substituted with one or more of the following groups : hydroxy, amino, carboxy, nitro, cyano, aryl, lower alkoxy, lower alkylthio or lower alkylen, preferably one or two substituents, $R^4$ and $R^5$ each represent hydrogen or $R^4$ together with $R^5$ represents straight or branched lower alkylen which may be substituted with one or more of the following groups : hydroxy, hydroxymethyl, amino, carboxy, nitro, cyano, aryl, lower alkoxy, lower alkylthio or lower alkylen, and which alkylen moiety may be interrupted by oxygen, preferably one or two substituents, or $R^4$, and $R^5$ together with the moiety

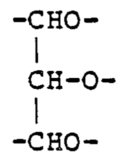

from formula I represents a carbohydrate moiety wherein — apart from one hydroxy group — the hydroxy groups, if desired, are acetalized or ketalized, which is characterized by treating an alcohol of the general formula II

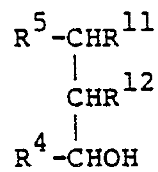

(II)

wherein $R^4$ and $R^5$ are as stated above, $R^{11}$ and $R^{12}$ each represents hydroxy, or $R^{11}$ together with $R^{12}$ represents a moiety of the general formula VI

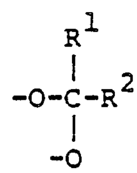

(VI)

wherein $R^1$ and $R^2$ are as stated above, with a carboxylic acid or a carboxylic acid ester of the general formula IV

$$R^3COOR^6 \qquad (IV)$$

wherein $R^3$ is as stated above, and $R^6$ represents hydrogen or has one of the meanings stated for $R^1$, above, or represents a group of the general formula V

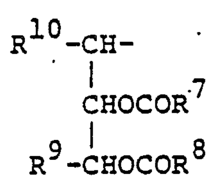

(V)

wherein $R^9$ and $R^{10}$ have the meanings stated for $R^4$ and $R^5$, above, and $R^7$ and $R^8$ are the same or different and each has one of the meanings stated for $R^3$, above, in the presence of an enzyme catalyzing esterification and, in case $R^{11}$ and $R^{12}$ each are hydroxy, in the presence of a carbonyl compound of the general formula III

$$R^1—CO—R^2 \qquad (III)$$

wherein $R^1$ and $R^2$ each are as stated above, and an agent catalyzing formation of acetal and ketal under dehydrating conditions.

According to one embodiment of this invention, $R^4$ and $R^5$ are each hydrogen. According to another embodiment of this invention, $R^4$ and $R^5$ together with the moiety

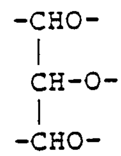

from formula I represents a carbohydrate moiety wherein — apart from one hydroxy group — the hydroxy groups, if desired, are acetalized or ketalized.

The carbonyl compound of formula III may be an alifatic ketone, for example, acetone. The carboxylic acid ester of formula IV may be a naturally occurring triglyceride.

The group $R^3$ may be a fatty acid residue containing from 8 to 24 carbon atoms, and, preferably, the fatty acid is palmitic acid, oleic acid, lauric acid or pelargonic acid.

Preferably, the groups $R^1$ and $R^2$ are hydrogen and lower alkyl, for example, methyl, ethyl or propyl. Preferably, the group $R^3$ is straight chain alkyl with 1-23 carbon atoms. The term « lower alkyl » herein designates alkyl containing not more than 6 carbon atoms. Preferably, aryl is phenyl. Hence, aryl (lower alkyl) is, preferably, phenyl (lower alkyl), most preferred benzyl and phenethyl. Lower alkoxy is, preferably, methoxy and ethoxy. Lower alkylthio is, preferably, methylthio and ethylthio. Lower alkylene is, preferably, methylene, ethylene and n-propylene. The group $R^6$ is preferably different from hydrogen.

Examples of preferred alcohols of the general formula IIb are as follows :

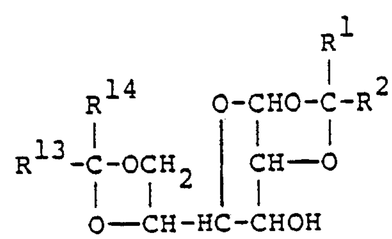

(IIc)
(glucose)

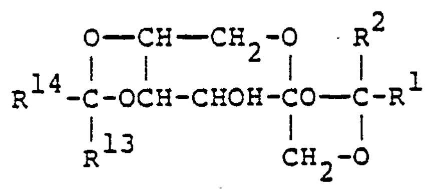

(IId)
(fructose)

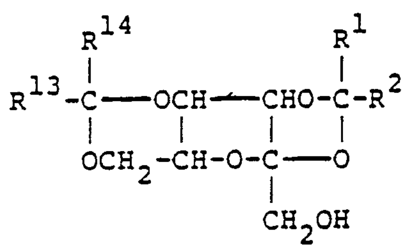

(IIe)
(sorbose)

wherein $R^1$ and $R^2$ are as defined above, and $R^{13}$ and $R^{14}$ are the same or different each representing hydrogen, alkyl, lower alkoxy, aryl or aryl (lower alkyl) each of which may be substituted with one or more of the following groups : hydroxy, amino, carboxy, nitro, cyano, aryl, lower alkoxy, lower alkylthio or lower alkylen, preferably one or two substituents. In a preferred embodiment, $R^1$, $R^2$, $R^{13}$ and $R^{14}$ are the same and preferably methyl. Furthermore, alcohols of formula II wherein $R^4$ and $R^5$ together with the moiety

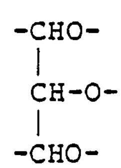

from formula I does not represent a corbohydrate moiety, are preferred.

A preferred embodiment of this invention relates to a process for preparing carboxylic acid esters of ketalized or acetalized monosaccharides or carboxylic acid esters of ketalized or acetalized glycerol,

characterized by treating a ketalized or acetalized monosaccharide or glycerol with a carboxylic acid or carboxylic acid ester in the presence of an enzyme catalyzing esterification.

A utility of the invention concerns preparation of monoglycerides of the general formula VII

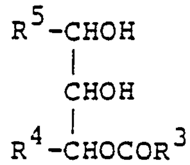

wherein $R^3$ is as defined above and $R^4$ and $R^5$ each is hydrogen. These compounds may readily be prepared according to the process of this invention, for example, by treating, as visualized in Scheme 1, below, the corresponding triglyceride of formula IVa with glycerol (formula IIf) and the appropriate carbonyl compound of formula IIIa in a solvent like hexane and in the presence of p-toluenesulfonic acid and a lipase at the boiling point of hexane which will serve both as a solvent for the reaction mixture and as a solvent for azeotropic removal of water generated in the course of the reaction. The ketalyzed monoglyceride of formula Ia can easily be isolated from the reaction mixture, for example by evaporation of the solvent after removal of the applied catalysts. Compounds of formula Ia may subsequently be converted into monoglycerides of formula VIIa, c.f. Scheme 1, for example, in a gentle acid medium, or it may be condensed with hydroxy acids or amino acids in a manner exemplified in Scheme 1 by conversion of compounds of formula Ia into the lactic acid ester of the free monoglycerides of formula IX.

Scheme 1

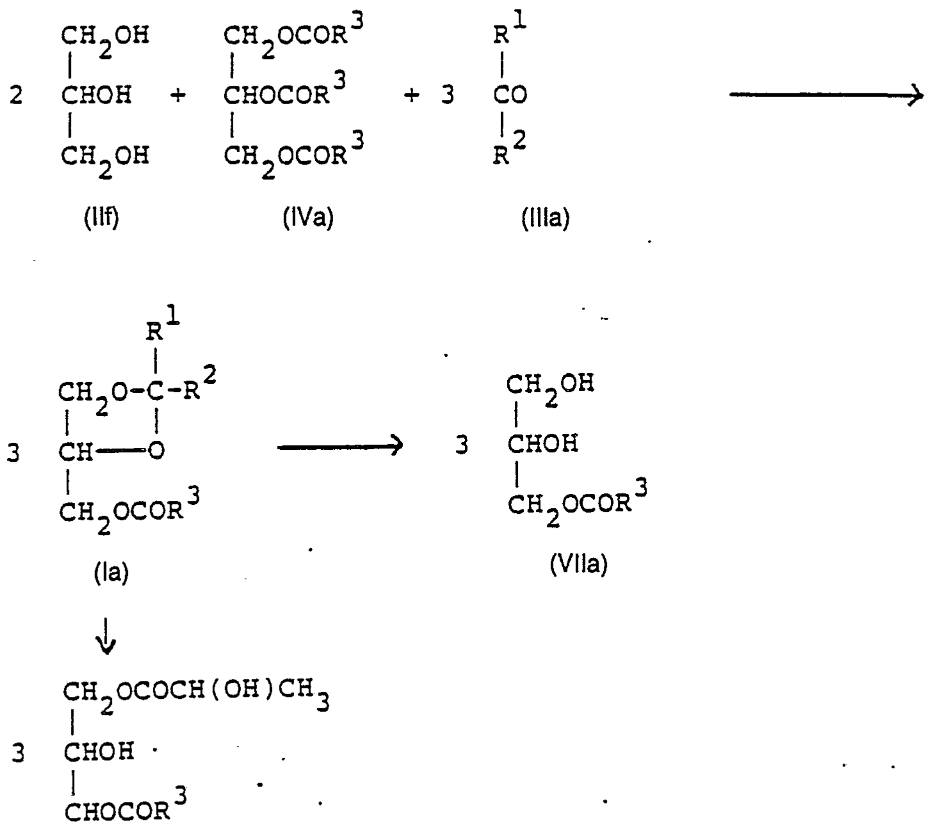

(IIf) (IVa) (IIIa)

(Ia) (VIIa)

(IX)

wherein $R^1$, $R^2$ and $R^3$ are as stated above.

Another utility of the invention relates to preparation of fatty acid esters of carbohydrates having the above formula VII which may be prepared in a manner analogous to the exemplified preparation of monoglycerides. Compounds of this nature which may thus be prepared according to the process of this invention can be utilized, for example, as detergents and emulsifying agents.

The features disclosed in the foregoing description and in the following examples and claims may,

both separately and in any combination thereof, be material for realising the invention in diverse forms thereof.

The process of this invention is further illustrated by the following examples which, however, are not construed as limiting. The examples illustrate some preferred embodiments of the invention. The letters TM after a name indicate that it is a trade mark.

Example 1

Diethylketone (7 ml), tributyrin (19.6 ml) and glycerol diethylketal (21.2 g) were dissolved in hexane (75 ml) and transferred to a Dean-Stark apparatus for azeotropic removal of water.

The reaction mixture was refluxed for one hour to remove water present in the reactants whereupon p-toluenesulfonic acid (1.25 g) and an immobilized Mucor miehei lipase prepared as described in Danish patent application No. 4167/84 and available from Novo Industri A/S (60 mg) were added to the reaction mixture. The reaction mixture was refluxed for 24 hours, an additional portion of lipase was added, and refluxing was finally continued for another 24 hours. The reaction mixture was analyzed by gas liquid chromatography (hereinafter GLC) using a Carbowax™ column and it was shown to contain the monobutyric acid ester of glycerol diethyl ketal in a yield close to 90 % based on the quantity of the applied tributyrin.

Example 2

1,2 : 5,6-di-O-isopropylidene-D-glucofuranose was dissolved in phosphate buffer (100 mM, pH 6.0) to provide a 3 % solution of the derivatised glucose. Methyl butyrate (3 equivalents) was added to 25 ml of this solution followed by the addition of 25 ml of hexane. Finally 200 mg of a freeze dried powder of Candida lipase was added to the reaction mixture which was subsequently stirred vigorously at 30 °C. The progress of the reaction was monitored by HPLC using a LiChrosorb-$NH_2$ column, acetonitrile/water as eluent and a refractive index detection system. After 24 hours the yield of 4-(1,2 : 5,6-di-O-isopropylidene-D-glucofuranosyl) butyrate was found to be 10 % and the product was isolated by chromatography on silica gel.

Example 3

1,2 : 5,6-di-O-isopropylidene-D-glucofuranose was esterified with decanoic acid using decanoic acid methyl ester as acyl doner and the lipase isolated from a strain of Mucor miehei in a manner analogous to the one described in Example 2. The yield of 4-(1,2 : 5,6-di-O-isopropylidene-D-glucofuranosyl) decanoate was 5 %.

Example 4

1,2 : 4,5-di-O-isopropylidene-D-fructopyranose was esterified in a manner analogous to the procedure described in Example 3. The yield of 3-(1,2 : 4,5-di-O-isopropylidene-D-fructopyranosyl) decanoate was 10 %.

Example 5

2,3 : 4,6-di-O-isopropylidene-D-sorbofuranose was esterified using methyl butyrate as acyl doner and hog pancreas lipase as the transacylating reagent in a manner analogous to the procedure described in Example 1. The yield of 1-(2,3 : 4,6-di-O-isopropylidene-D-sorbofuranosyl) butyrate was 8 %.

Example 6

1,2 : 5,6-di-O-isopropylidene-D-glucofuranose was esterified in a two-phase system using toluene as the organic phase and a 4 M sodium chloride solution in phosphate buffer (100 mM pH 6) as the aqueous phase. The acyl doner applied was tri-sterin and the transacylating agent the immobilized Mucor miehei lipase. Otherwise, the synthesis was performed according to the procedure outlined in Example 1. The yield of 4-(1,2 : 5,6-di-O-isopropylidene-D-glucofuranosyl) stearate was 20 %.

Example 7

1,2 : 5,6-di-O-isopropylidene-D-glucofuranose was dissolved in a mixture of hexane and benzene (3 : 7 (v/v), 50 mM) together with methyl decanoate (50 mM). The reaction mixture was stirred vigorously while a freeze dried powder of Nocardiopsis lipase was added to the reaction mixture. Stirring was continued for 18 hours whereupon the reaction mixture was analyzed as indicated in Example 1, the yield of 3-(1,2 : 5,6-di-O-isopropylidene-D-glucofuranyl) decanoate being 15 %.

Example 8

1,2 : 5,6-di-O-isopropylidene-D-glucofuranose was esterified in a manner analogous to that described in Example 7 using toluene as solvent. The yield of 3-(1,2 : 5,6-diisopropylidene-D-glucofuranosyl) decanoate was 20 %.

Example 9

1,2 : 5,6-di-O-isopropylidene-D-glucofuranose was esterified as indicated in Example 7 by using benzene as the organic solvent. The yield of 3-(1,2 : 5,6-diisopropylidene-D-glucofuranosyl) decanoate was 10 %.

Example 10

1,2 : 5,6-di-O-isopropylidene-D-glucofuranose was esterified as indicated in Example 7 using hexane/acetone (9 : 1 (v/v)) as solvent. The yield of 3-(1,2 : 5,6-diisopropylidene-D-glucofuranosyl) decanoate was 15 %.

Example 11

1,2 : 5,6-di-O-isopropylidene-D-glucofuranose was esterified with methyl palmitate in a manner analogous to that described in Example 2 the lipase from a species of Pseudomononas being used as catalyst. A yield of 3-(1,2 : 5,6-diisopropylidene-D-glucofuranosyl) palmitate of 15 % was achieved.

Example 12

Application of freeze dried lipase from Nocardiopsis as described in Example 11 served to provide 3-(1,2 : 5,6-diisopropylidene-D-glucofuranosyl) palmitate in a yield of 25 %.

Example 13

Application of a freeze dried powder of Serratia lipase analogously to the manner described in Example 11 provided 3-(1,2 : 5,6-diisopropylidene-D-glucofuranosyl) palmitate in a yield of 15 %.

Example 14

Application of a freeze dried powder of Geotricium lipase analogously as described in Example 11 served to provide 3-(1,2 : 5,6-diisopropylidene-D-glucofuranosyl) palmitate in a yield of 10 %.

The formulae IIc, IId and IIe mentioned above are represented on the following page.

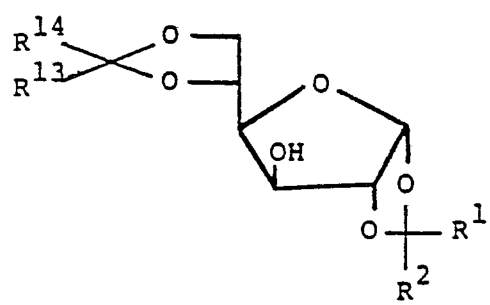

(IIc)
(glucose)

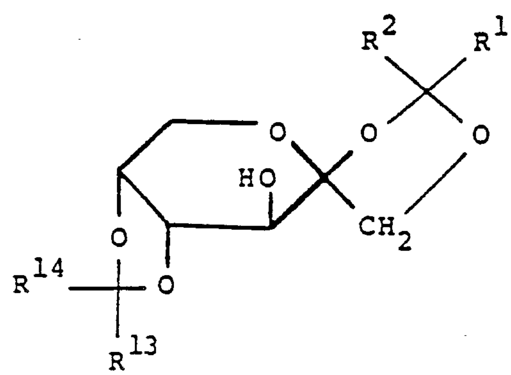

(IId)
(fructose)

(Continued)

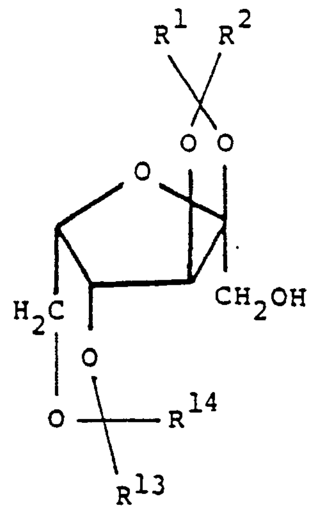

(IIe)
(sorbose)

## Claims

1. Process for preparing compounds of the general formula I

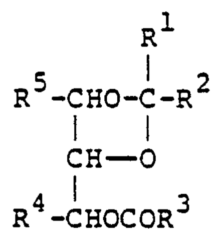

(I)

wherein $R^1$, $R^2$ and $R^3$ are the same or different representing hydrogen, alkyl, aryl or aryl (lower alkyl) each of which may be substituted with one or more of the following groups: hydroxy, amino, carboxy, nitro, cyano, aryl, lower alkoxy, lower alkylthio or lower alkylen, preferably one or two substituents, $R^4$ and $R^5$ each represent hydrogen or $R^4$ together with $R^5$ represents straight or branched lower alkylen which may be substituted with one or more of the following groups: hydroxy, hydroxymethyl, amino, carboxy, nitro, cyano, aryl, lower alkoxy, lower alkylthio or lower alkylen, and which alkylen moiety may be interrupted by oxygen, preferably one or two substituents, or $R^4$ and $R^5$ together with the moiety

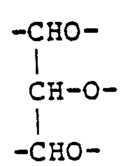

from formula I represents a carbohydrate moiety wherein — apart from one hydroxy group — the hydroxy groups are acetalized or ketalized, characterized by treating an alcohol of the general formula II

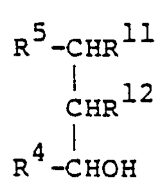

(II)

wherein $R^4$ and $R^5$ are as stated above, $R^{11}$ and $R^{12}$ each represents hydroxy, or $R^{11}$ together with $R^{12}$ represents a moiety of the general formula VI

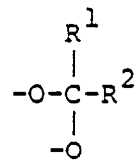 (VI)

wherein $R^1$ and $R^2$ are as stated above, with a carboxylic acid or a carboxylic acid ester of the general formula IV

$$R^3COOR^6 \quad (IV)$$

wherein $R^3$ is as stated above, and $R^6$ represents or has one of the meanings stated for $R^1$, above, or represents a group of the general formula V

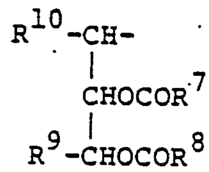 (V)

wherein $R^9$ and $R^{10}$ have the meanings stated for $R^4$ and $R^5$, above, and $R^7$ and $R^8$ are the same or different and each has one of the meanings stated for $R^3$, above, in the presence of an enzyme catalyzing esterification and, in case $R^{11}$ and $R^{12}$ each are hydroxy, in the presence of a carbonyl compound of the general formula III

$$R^1—CO—R^2 \quad (III)$$

wherein $R^1$ and $R^2$ each are as stated above, and an agent catalyzing formation of acetal and ketal under dehydrating conditions.

2. Process, according to Claim 1, for preparing carboxylic acid esters of ketalized or acetalized monosaccharides or carboxylic acid esters of ketalized or acetalized glycerol, characterized by treating a ketalized or acetalized monosaccharide or glycerol with a carboxylic acid or carboxylic acid ester in the presence of an enzyme catalyzing esterification.

3. Process, according to Claim 1 or 2, wherein said enzyme is a lipase.

4. Process, according to Claim 1 or 2, wherein said enzyme is an esterase.

5. Process, according to any one of the preceding claims, wherein said enzyme is an immobilized enzyme.

6. Process, according to any one of the preceding claims, wherein the process is carried out in a solvent such as a hydrocarbon, preferably hexane or pentane.

7. Process, according any one of the preceding claims, wherein the monosaccharide is glucose, galactose, fructose or sorbose.

**Patentansprüche**

1. Verfahren zum Herstellen von Verbindungen der allgemeinen Formel I

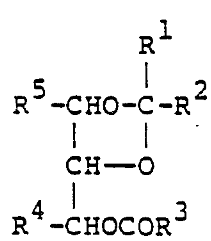 (I)

in der $R^1$, $R^2$ und $R^3$ identisch oder verschieden sind, wobei sie jeweils Wasserstoff, Alkyl, Aryl oder Aryl (niederalkyl) darstellen, von denen jedes mit einer oder mehreren der folgenden Gruppen substituiert sein kann : Hydroxy, Amino, Carboxy, Nitro, Cyano, Aryl, Niederalkoxy, Niederalkylthio oder Niederalkylen, vorzugsweise ein oder zwei Substituenten, $R^4$ und $R^5$ jeweils Wasserstoff darstellen oder $R^4$ zusammen mit $R^5$ geradkettiges oder verzweigtes Niederalkylen darstellt, das mit einer oder mehreren der folgenden Gruppen substituiert sein kann : Hydroxy, Hydroxymethyl, Amino, Carboxy, Nitro, Cyano, Aryl, Niederalkoxy, Niederalkylthio oder Niederalkylen, und wobei die Alkylenkomponente von Sauerstoff unterbrochen sein kann, vorzugsweise ein oder zwei Substituenten, oder $R^4$ und $R^5$ zusammen mit der Komponente

```
-CHO-
 |
 CH-O-
 |
-CHO-
```

aus Formel I eine Kohlehydratkomponente darstellt, in der — abgesehen von einer Hydroxygruppe — die Hydroxygruppen acetalisiert oder ketalisiert sind, dadurch gekennzeichnet, daß ein Alkohol der allgemeinen Formel II

```
R5-CHR11
    |
    CHR12
    |
R4-CHOH
```
(II)

in der $R^4$ und $R^5$ die oben angegebene Bedeutung haben, $R^{11}$ und $R^{12}$ jeweils Hydroxy darstellen oder $R^{11}$ zusammen mit $R^{12}$ eine Komponente der allgemeinen Formel VI

```
    R1
    |
-O-C-R2
    |
   -O
```
(VI)

in der $R^1$ und $R^2$ die oben angegebene Bedeutung haben, darstellt, mit einer Carbonsäure oder einem Carbonsäureester der allgemeinen Formel IV

$$R^3COOR^6 \qquad (IV),$$

in der $R^3$ die oben angegebene Bedeutung hat und $R^6$ Wasserstoff darstellt oder eine der oben für $R^1$ angegebenen Bedeutungen besitzt oder eine Gruppe der allgemeinen Formel V

```
R10-CH-
     |
     CHOCOR7
     |
R9-CHOCOR8
```
(V)

in der $R^9$ und $R^{11}$ die oben für $R^4$ und $R^5$ angegebenen Bedeutungen besitzen und $R^7$ und $R^8$ identisch oder verschieden sind und jeweils eine der oben für $R^3$ angegebenen Bedeutungen besitzen, darstellt, in Gegenwart eines esterifizierungskatalysierenden Enzyms und, falls $R^{11}$ und $R^{12}$ jeweils Hydroxy sind, in Gegenwart einer Carbonylverbindung der allgemeinen Formel III

$$R^1—CO—R^2 \qquad (III),$$

in der $R^1$ und $R^2$ die oben angegebenen Bedeutungen haben, und eines Agens, das die Bildung von Acetal und Ketal unter dehydratisierenden Bedingungen katalysiert, behandelt wird.

2. Verfahren nach Anspruch 1 zum Herstellen von Carbonsäureestern ketalisierter oder acetalisierter Monosaccharide oder Carbonsäureestern ketalisierten oder acetalisierten Glycerins, dadurch gekennzeichnet, daß ein ketalisiertes oder acetalisiertes Monosaccharid oder Glycerin mit einer Carbonsäure oder einem Carbonsäureester in Gegenwart eines esterifizierungskatalysierenden Enzyms behandelt wird.

3. Verfahren nach Anspruch 1 oder 2, dadurch gekennzeichnet, daß besagtes Enzym eine Lipase ist.

4. Verfahren nach Anspruch 1 oder 2, dadurch gekennzeichnet, daß besagtes Enzym eine Esterase ist.

5. Verfahren nach einem der vorangehenden Ansprüche, dadurch gekennzeichnet, daß besagtes Enzym ein immobilisiertes Enzym ist.

6. Verfahren nach einem der vorangehenden Ansprüche, dadurch gekennzeichnet, daß das Verfahren in einem Lösungsmittel wie einem Kohlenwasserstoff, vorzugsweise Hexan oder Pentan, durchgeführt wird.

7. Verfahren nach einem der vorangehenden Ansprüche, dadurch gekennzeichnet, daß das Monosaccharid Glukose, Galaktose, Fruktose oder Sorbose ist.

**Revendications**

1. Procédé de préparation de composés de formule générale I

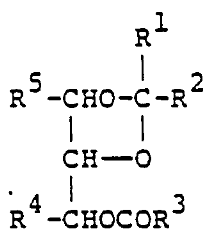

(I)

dans laquelle $R^1$, $R^2$ et $R^3$ sont identiques ou différents, chacun représentant de l'hydrogène, un alkyle, un aryle ou un aryl (alkyle inférieur) dont chacun peut être substitué par un ou plusieurs des groupes suivants : hydroxy, amino, carboxy, nitro, cyano, aryle, alcoxy inférieur, alkylthio ou alkylène inférieur, de préférence par un ou deux substituants, $R^4$ et $R^5$ représentent chacun l'hydrogène ou $R^4$ représente conjointement avec $R^5$ un alkylène inférieur droit ou ramifié qui peut être substitué par un ou plusieurs des groupes suivants : hydroxy, hydroxyméthyle, amino, carboxy, nitro, cyano, aryle, alcoxy inférieur, alkylthio inférieur ou alkylène inférieur, ladite partie alkylène pouvant être interrompue par de l'oxygène, de préférence par un ou deux substituants, ou $R^4$ et $R^5$ représentent, ensemble avec le fragment

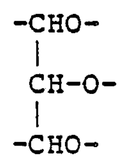

de formule I, un fragment d'hydrate de carbone où, mis à part un groupe hydroxyle, les groupes hydroxyles sont acétalisés ou cétalisés, caractérisé par le traitement d'un alcool de formule générale II

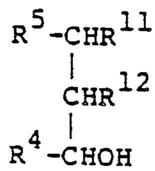

(II)

dans laquelle $R^4$ et $R^5$ sont tels que définis précédemment, $R^{11}$ et $R^{12}$ représentent chacun un hydroxy, ou $R^{11}$ représente conjointement avec $R^{12}$ un fragment de formule générale VI

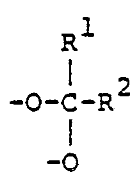

(VI)

dans laquelle $R^1$ et $R^2$ sont tels que définis précédemment, avec un acide carboxylique ou un ester d'acide carboxylique de formule générale IV

$$R^3COOR^6 \qquad (IV)$$

où $R^3$ est tel que défini plus haut, et $R^6$ représente de l'hydrogène ou possède une des significations indiquées à propos de $R^1$ ci-dessus, ou représente un groupe de formule générale V

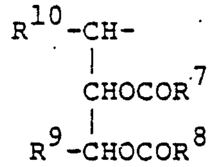

dans laquelle $R^9$ et $R^{10}$ ont les significations indiquées à propos de $R^4$ et $R^5$ ci-dessus et $R^7$ et $R^8$ sont identiques ou différents et possèdent chacun l'une des significations indiquées à propos de $R^3$ ci-dessus, en présence d'une enzyme catalysant l'estérification et, dans le cas où chacun des $R^{11}$ et $R^{12}$ est un hydroxyle, en présence d'un composé carbonylé de formule générale III

$$R^1—CO—R^2 \qquad (III)$$

dans laquelle $R^1$ et $R^2$ sont chacun tels que définis précédemment, et d'un agent catalysant la formation d'un acétal et d'un cétal dans des conditions de déshydratation.

2. Procédé selon la revendication 1 pour la préparation d'esters d'acides carboxyliques et de monosaccharides cétalisés ou acétalisés ou d'esters d'acides carboxyliques et de glycérol cétalisé ou acétalisé, caractérisé par le traitement d'un monosaccharide ou de glycérol cétalisé ou acétalisé avec un acide carboxylique ou un ester d'acide carboxylique en présence d'une enzyme catalysant l'estérification.

3. Procédé selon la revendication 1 ou 2, dans lequel l'enzyme est une lipase.

4. Procédé selon la revendication 1 ou 2, dans lequel l'enzyme est une estérase.

5. Procédé selon l'une quelconque des revendications précédentes, dans lequel ladite enzyme est une enzyme immobilisée.

6. Procédé selon l'une quelconque des revendications précédentes, dans lequel le procédé est conduit dans un solvant tel qu'un hydrocarbure, de préférence l'hexane ou le pentane.

7. Procédé selon l'une quelconque des revendications précédentes, dans lequel le monosaccharide est le glucose, le galactose, le fructose ou le sorbose.